# EUROPEAN PATENT APPLICATION

(11) **EP 3 249 041 A1**
(43) Date of publication of application: **29.11.2017**
(21) Application number: 14907029.4
(22) Date of filing: 24.11.2014
(51) Int. Cl.: C12N 1/20

(54) **CULTURE MEDIUM ALLOWING THE GROWTH OF THE BACTERIUM PISCIRICKETTSIA SALMONIS**

(71) Applicant: Pontificia Universidad Católica De Valparaíso, Valparaíso (CL)
(72) Inventor: ALTAMIRANO, Claudia, Valparaiso (CL); MARTíNEZ, Irene, Valparaiso (CL); MARSHALL, Sergio, Valparaiso (CL); HENRíQUEZ, Vitalia, Valparaiso (CL); GÓMEZ, Fernando, Valparaiso (CL); FUENTEALBA, Pablo, Valparaiso (CL)
(74) Representative: Valea AB
(86) International application number: PCT/CL2014/000065
(87) International publication number: WO 2016/082050

(57) **Abstract**

The invention relates to a novel cell-free liquid culture medium which allows the growth of the bacterium *Piscirickettsia salmonis* in a minimum period of time, by means of the nutritional supply of defined minimum components with concentrations adjusted to the demand therefor, allowing a previously unachieved maximum concentration of biomass with a high yield to be obtained. The bacterial phenotype obtained in the defined cell-free medium preserves the levels of virulence observed in traditional culture systems. This technology therefore supports the development of a novel process which is faster and more economical than the current systems, generating a product of equal or better quality, in order to obtain a biomass required for both oral and injectable vaccine formulations.

## Description

### FIELD OF THE INVENTION

The present invention falls within the biotechnology field and corresponds to a novel cell-free liquid culture medium which allows the growth of the bacterium *Piscirickettsia Salmonis* in a minimum period of time, by means of the nutritional supply of defined minimum components with concentrations adjusted to the demands therefor, allowing a previously unachieved maximum concentration of biomass with a greater yield to be obtained. The bacterial phenotype obtained in the defined cell-free medium preserves the levels of virulence observed in traditional culture systems. This technology therefore supports the development of a novel process which is faster and more economical than the current systems in order to obtain the required biomass for both oral and injectable vaccine formulations.

### STATE OF THE ART

The high spatial expansion and the intensiveness of salmon cultures have favoured the propagation of illnesses that have seriously impaired production. The confined space of the culture sectors, the slow water rechange of water in the canal systems, the nearness between culture centers, the high density of fish cultured by the cage system and the immunological repression produced in the fish submitted to the preceding conditions explain to a great extent the ethiological explosive episodes occurring in the salmon culture. Among pathogenic organisms in a high incidence on aquiculture we find a virus, bacteria, some fungi and parasites; the following can be underscored: the infectious Pancreatic Necrosis Virus, IPM, the Salmon-ISAV Anaemia Virus (Kibenge, F.S.B., Godoy, M.G., Fast, M., Workenhe, S. & Kibenge, M.J.T. (2012). Countermeasures against viral diseases of farmed fish. Antiviral Research, 95 (3), 257.281); the Saprolegnia parasitica (Zaror, L., Collado, L., Bohle, H., Landstroke, E., Montaña, J. & Avendaño, F. (2004). Saprolegnia parasitica in salmon and trout of the South of Chile. Archivos de Medicina Veterinaria, 36(1), 71-787*)*, as well as Gram-negative bacteria, such as Aeromonas salmonicida (Balcázar, J.L, Vendrell, D., de Bias, I., Ruiz-Zarzuela, I., Gironés, O. P. & Múzquiz. J.L., 2007). Quatitative detection of Aeromonas salmonicida in salmon and trout fish tissue by real-time PCR using self-quenched, fluorogenic primers. Journal of Medical Microbiology, 56 (3), 323-328); Vibrio ordalii (Bohle, H., Kjetil, F., Bustos, P., Riofrío, A. and Peters, C. (2007). An atypical phenotype of *Vibrio ordalii,* a high pathogenic bacteria isolated from Atlantic salmon cultivated in the South of Chile sea coast). Archivos de Medicina Veterinaria, 39(1), 43-52). Flavobacterium columnare (Bader, J.A. & Shotts Jr., E.B. (1998). Identification of Flaviobacterium and Flexibacter species by species-specific polymerase chain reaction primers to the 16S ribosomal ARN gene. Journal of Aquatic Animal Health, 10(4), 311-319) and recently the appearance of the *Francisella piscicida bacteerium,* formerly known as UA2 (Birbeck, T.H., Bodevik, M., Frøystad, M.K. & Baklien, A. (2007), Identification of Francisella sp. from Atlantic salmon to Salmo salar L. in Chile. Journal of Fish Diseases, 30(8), 505-507), in addition to the Gram-positive *Renibacterium salmoninarum* (Sanders. J.E. & Manuel Jose, B.R. (1986). Evidence by the florescent antibody test for the occurrence of Renibacterium samloninarum among salmonid fish in Chile, Journal of Wild Life Diseases, 22 (2), 255-257). However, and undoubtedly the pathogen of highest relevance that has demonstrated a greatest aggressiveness and persistence since its appearance is the *Piscirickettsia salmonis*) bacteria (Fryer, J.L. & Hedrick, R.P. (2003). Piscirickettsia salmonis: a Gram-negative intracellular bacterial pathogen of fish, Journal of Fish Diseases, 26(5), 251-262; Marshall, S.H., Conejeros, P., Zahr, M., Olivares, J., Gomez, F., Cataldo, P and Henríquez, V. (2007), Immunological characterization of a bacterial protein isolated from salmonid fish naturally infected with Piscirickettsia salmonis, Vaccine 25(11), 2095-2102. doi:http://dx.doi.org/10.1016/j.vaccine. 2006,11.035), which is attributed a 94 and 88% of the mortalities caused by Atlantic salmon and rainbow trout pathologies, respectively, during 2013 in Chile (SERNAPESCA, 2014). Salmoniculture Sanitary Report in marine centers, year 2013.

http://www.sernapesca.cl/index.php?option=com remositorv&ltemid=246&func=fileinfo &id=8 014). In Chile, Salmo Ricketsial Syndrome (SRS) caused by the *Piscirickettsia salmonis* bacteria has generated economic losses in excess of US$150 million due to the appearance of constant outbreaks; it has been estimated that the SRS-associated mortality is close to 15% of the national industry biomass (Sanchez, V., (2003), Piscirickettsiosis: The challenge of maintaining control. Aquanoticias Internacional 80: 57-62.) The disease has also been recorded in countries like Scotland, Ireland, Norway and the Canada Pacific Coast (Kusyk, M.A., Thorson, J.C. & Kay W.W. (1996), Antigenic characterization of the salmonid pathogen Piscirickettsia salmonis, Infection and Immunity, 64(12), 5205-5210), where economic losses have been quite considerable, but never at the level in which production in Chile was affected (Almendras, F.E., Fuentealba, I.C., Jones, S.R. M., Markham, F. & Spangler, E. (1997). Experimental infection and horizontal transmission of Piscirickettsia salmonis in freshwater-raised Atlantic salmon, Salmo salar L., Journal of Fish Diseases, 20(6), 409-418). In this manner, not only salmonid species have been affected by the presence of *P. salmonis,* but the presence of this pathogen has also been detected in specimens of White Corbina *(Atractoscion nobilis)* in the South coast of California (Arkush, K.D., McBride, A.M., Mendonça, H.L., Okihiro, M.S., Andree, K.B., Marshall, S., Hedrick, R.P. (2005). Genetic characterization and experimental pathogenesis of Piscirickettsia salmon isolated from white seabass, Attractoscion nobilis, Diseases of Aquatic Organisms, 63(2), 139-149). Specimens of European lubina - a variety of seabass - *(Dicentrarchus labrax)* in Greece have been affected by a pathogen very similar to *P.* Salmonis (Athanassopoulou, F., Groman, D., Prapas, T. & Sabatakou, O. (2004), Pathological and epidemiological observations on rickettsiosis in cultured sea bass (Dicentrarchus labrax L.) from Greece Journal of Applied Ichthyology, 20(6), 525-529); likewise it has been reported that in Hawai the populations of Tilapia (*Oreochromis mossambicus* and *Sarotherodon melanotheron*) both in free life and under cultivation, have suffered a disease of the Piscirickettsiosis type (Mauel, M.J., Miller, D.L., Frazier, K., Liggett, A.D., Styer, L., Montgomery-Brok, D. & Brock, J. (2003). Characterization of a Piscirickettsiosis-like disease in Hawaiian tilapia. Diseases of Aquatic Organisms, 53(3), 249-255); this suggests the expansion of this agent to other fish species of commercial importance (Marshall *et al*, 2007). By way of control measures for the pathologies expansion, new productive measures have been formulated and adopted in Chile that propitiate better and more stringent handling practices in cultivation centers, since a production inflexion point was produced caused to the ISAV bursting in 2008. Thus, the preventive and monitoring systems acquired higher importance in salmon culture. Within the adopted preventive strategies, the intensification should be mentioned of the use of stimulants of the immune system as well as the immunological conditioning faced to higher incidence pathologies. In the latter point, vaccines play a significant role, inasmuch as they immunologically stimulate fish faced to existing and persistent pathologies in the culture systems. For this reason, the vaccine producing veterinary industry has been proposed to search efficient and low-cost alternatives that permit to support the demand and respond with efficient products to the aquiculture industry. Particularly, vaccines against *Piscirickettsia salmonis* present a difficult and operationally complicated productive processes, because the system involves several stages and extensive cultivation periods. The latter is mainly due to the process implemented for production of P. *salmonis* biomass required for the vaccine generation, which consists of the culture of a host cellular line that should be nourished and next proliferated up to a determined density and then infected with *P. salmonis.* This process commences the *P. salmonis* cultivation stage inside the host cell. Upon completion of *P. salmonis* cultivation, the different cellular types should be separated; this represents a technical difficulty that directly influences the vaccine quality. This is the current general procedure for obtaining *P. salmonis* biomass and to produce the vaccines used for controlling the disease and the immunological stimulation of SRS fish. From the industrial point of view, this process in use for cultivation of *P. salmonis* constitutes the only unitary operation currently scalable at the industrial level, which has permitted to obtain a reliable biomass for the production of vaccines against the pathogen.

As concerns cells free cultivation, in 2008 Mauel *et al* (Mauel, M.J., Ware, C. & Smith, P.A. (2008), Culture of Piscirickettsia salmonis on enriched blood agar. Journal of Veterinary Diagnostic Investigation (20(2), 213-214, doi:10.1177/10406387080021000211) as well as Mikalsen *et al.* (Mikalsen, J., Skjaervic, O., Wiik-Nielsen J., Wasmuth, M.A., Colquhoun D.J. (2008), Agar culture of Piscirickettsia salmonis, a serious pathogen of farmed salmonid and marine fish, FEMS Microbiol. Lett. 2008. Jan; 278(1):43-7) independently achieved the axenic cultivation (host-cells free) of this microorganism in a solid and liquid medium containing lamb blood and cysteine. In this case, however, the difficulty is still present that the cultivation at the industrial level, even when they correspond to axenic media, bearing in mind that these media are hardly scalable, beating in mind that in this condition a low bacterial density is obtained as compared with the eukariont (host) cells infection systems.

In connection with patents applications currently filed for SRS prevention, some exist that relate to the formulation of vaccines, both from bacterines and antigenic recombinant proteins.

The US Patent 7.707.970 of Novartis AG proposes the use of a vaccine from a non-virulent *Anthrobacter* (RENOGEN) or antigenic *Anthrobacter* for SRS prevention. Vaccines based on *Anthrobacter were* originally devised for preventing kidney bacterial disease, although it is deemed that they would have the capacity of preventing SRS, a result that has not yet been proven.

The US Patent 6.887.989 expresses that DNA sequences and *Piscirickettsia salmonis aminoacids* are available and have been also considered for formulating SRS vaccines.

The Chilean patent 50237 suggests that the nucleic acid fragment that codifies the 17 kda external surface of lipoprotein (OspA) of *Piscirickettsia salmonis* can be used as a vaccine for protecting poiquilotherm fish against *P. salmonis* infection.

Chilean patents 46351, 46115, 46227, 46059 indicate that different thermal shock proteins, such as HSAP10, HSP16 and HSP60 or ChaPs and HSP70 can be used as proteins that permit to generate an immune response against *Piscirickettsia salmonis* in vertebrates.

However, none of these patents, improves the production level and efficiency for the development of vaccines as expected by the industry, so it deserves find new and more efficient alternatives.

International Publication WO2008002152 reports a complex growth medium, based on yeast extract and casein. These substrates are composed of multiple molecules in a relative proportion and even indefinite, which makes unspecific the medium formulated to *Piscirickettsia salmonis* growth. Additionally, a third substrate (glycerol or sucrose or maltose) was evaluated for growth. The maximum optical density (OD) measured at 600 nm (OD₆₀₀ₙₘ) achieved by the formulations proposed herein reaches 2.85 at 144 hours of culture. Is also reported here in culture conditions without pH control is achieved an 0D_{600nm ∼}8 and in pH conditions controlled in 6.5 an OD_{600nm ∼}13 is achieved at 120 hours. These conditions were evaluated using the commercial medium SF900II™ of Life Technologies for Spodoptera frugiperda cell lines. The business environment SF900II™ has not released information on its formulation. It is therefore not possible to make approximations of the yields of the culture of *P. salmonis* in this medium, nor to derive a formulation therefrom.

International Publication WO2013084169, instead describes new free culture media blood for use in liquid and solid cultivation *Piscirickettsia salmonis* bacterium. Regarding the liquid culture medium of this document, 19 of the 20 amino acids are used existing in nature (Table 2), and a large number of inorganic salts and other components, making it economically not quite viable. In the same sense, the medium uses an amount not less (100ml) Fetal Bovine Serum (FBS), which, as is known in the prior art, has a number of vitamins, amino acids and growth factors (unquantified) that stimulate the growth of organisms, making it non-reproducible, increasing non-specificity as growth medium and also making expensive the preparation of a potentially scalable culture medium. It is also reported in the same publication that the growth of bacteria in colony forming units (CFU) optimal for use in vaccine production, is only achieved after 144 hours of culture.

The inventors of the present invention have successfully developed a scaleable culturing *P. salmonis* technology industrially consisting of three steps 1) propagation, 2) bioreactor culture and 3) recovery. The state of the art does not report culture media for *P. salmonis* evaluated scale bioreactor.

### SUMMARY OF THE INVENTION

The present invention relates to an essential minimum defined medium for culturing a bacterium belonging to genus *Piscirickettsia* such as *Piscirickettsia salmonis,* wherein it is said bacterium is grown in a free liquid medium of cells, with minimal components, associated with particular parameters, allows the growth of the bacteria in a shorter time, maintaining its virulence.

A related aspect of the invention is the specific production procedure of the bacterium of the genus *Piscirickettsia,* using the cell-free liquid medium.

Another aspect of the invention relates to the free liquid medium of cells corresponding to a defined minimum essential medium, giving biomass of bacteria belonging to the genus Piscirickettsia, said bacterium is characterized as stable in its properties virulence and infectivity.

Another aspect of the invention relates to the use of biomass of bacteria of the genus Piscirickettsia, in the production of one or more vaccine formulations comprising *Piscirickettsia.*

Another aspect of the invention relates to the use of bacteria of the genus *Piscirickettsia* for use in veterinary medicine.

Another aspect of the invention relates to the use of bacteria of the genus *Piscirickettsia* or a sub-unit of said bacterium in the manufacture of a medicament for the prevention of infections caused by bacteria of the genus *Piscirickettsia.*

The aim of the present invention is to provide an essential minimum culture medium, to obtain a maximum yield of biomass *Piscirickettsia,* maintaining its virulence and infectivity, with which it is possible to formulate vaccines for veterinary use, for preventing infections in fish with bacteria of the genus *Piscirickettsia.*

Another object of the present invention comprises culturing the bacterium *Piscirickettsia,* increased biomass, vaccine formulation and administration to fish of one or more vaccines according to the invention.

### DESCRIPTION OF THE FIGURES

FIGURE 1: Growth kinetics of *P. salmonis* MD1 expressed as biomass (mg/L) obtained in time (h).
FIGURE 2: Kinetics of growth of *P. salmonis* in MD1 expressed as cell density (N° cells/mL) obtained in time (h).
FIGURE 3: Kinetics of growth of P. salmonis in MD1 expressed as optical density (OD) measured at 600 nm, obtained at the time (h).
*FIGURE4**:* Growth kinetics of *P. salmonis* im MD2 expressed as biomass (mg/L) obtained in time (h).
FIGURE 5: Kinetics of growth of *P. salmonis* in MD2 expressed as cell density (N° cells/mL) obtained in time (h).
FIGURE 6: Kinetics of growth of *P. salmonis* in MD2 expressed as optical density (OD) measured at 600 nm, obtained at the time (h).
FIGURE 7: Stages of growing new technology in cell free defined medium for P. salmonis. (P) Propagation of P. salmonis flask level to generate the inoculum for the bioreactor culture. (CB) bioreactor culture. (R) Recovery of biomass by centrifugation.
FIGURE 8: Characteristics of the cultivation process of *P. salmonis* in cell line at industrial level. (P1) Propagation eukaryotic cell line in bioreactor cultivation. (P2) Propagation of *P. salmonis* in eukaryotic cell line for the infection process. (CB) Bioreactor culture of eukaryotic cell line. (I) Infection of eukaryotic cell line grown in bioreactor with *P. salmonis.* (R) Recovery in two stages of the bacterial biomass by centrifugation.
FIGURE 9: Cumulative mortality attributable to *P. salmonis* testing challenge in Salmo salar pre-smolt stage. (Control) not challenged with a harmless and injected solution to determine mortality fish management. The concentration of challenge from highest to lowest is: Direct> Dil-1> Dil-2> Dil-3.
FIGURE 10: Level of protection of vaccines formulated with *P. Samonis* biomass grown in MD-2, viewed as the cumulative mortality in trials of challenges with Salmo salar. (Control) non-vaccinated fish.

### DETAILED DESCRIPTION OF THE INVENTION

The inventors of the present invention have developed a culture technology consisting of three general steps: 1. Propagation; 2. Cultivation in the bioreactor; 3. Recovery.

The present invention relates to the specific formulation of a defined liquid medium for the cultivation of *P. salmonis,* based on the basic nutritional requirements of this bacterium liquid culture also has features such as low cost in its construction and is scalable to industrial level.

Prior to the propagation step is necessary to defrost the strain stored at -80°C and propagate it in culture flasks. This process involves cryopreservation, and activation of the bacteria of the genus *Piscirickettsia* such as EM-90, CI 95, Nor-92, ATL-4-91, SGLO-94, more specifically the LF-89 strain and variants of *P. salmonis* bacterium, used to inoculate the cultures with minimum essential medium of the invention.

Conserving, i.e cryopreservation strains of the bacterium of the genus *Piscirickettsia,* EM-90, CI-95, Nor-92, ATL-4-91, SGLO-94, more specifically the LF-89 strain is performed by generation of a cell bank for cryopreservation under low temperatures between -20 and -196°C, more specifically between -30° and - 100°C, more specifically between -50°C and 90°C, more specifically between -70°C and -80°C. The cryopreservative solution corresponds to a glycerol solution between 10% and 100%, between 20% and 100%, between 30% and 100%, between 40% and 100%, between 50% and 100%, between 60% and 100% , between 70% and 100%, between 80% and 100%, between 90% and 100% at 100% and molar sacarose (M) solution between 0.1M and 1M, between 0.2M and 0.9M, between 0.3M and 0.8M, between 0.4M and 0.7M, between 0.5M and 0.6M, preferably in a ratio of 1:1, 1:2, 1:3, more preferably 1:4, which has a pellet of *Piscirickettsia* genus of bacteria, such as *Piscirickettsia salmonis.* Thus, it provides a working stock with the strains described.

It should be noted that the storage process of *P. salmonis* was developed by the research group of the inventors of the present invention.

The activation is made by transferring bacteria of the *Piscirickettsia* cryopreserved genus, a complex medium in flasks as reported by Henriquez, M *et al.* (2013) (Henriquez, M., Gonzalez, E., Marshall, S.H, Henriquez V. Gomez, F., Martinez, I., & Altamirano, C. (2013). A novel liquid medium for the efficient growth of the Piscirickettsia salmonis salmonid pathogen and optimization of culture conditions PloS One, 8(9), e71830 doi:10.1371/journal.pone.0071830) corresponding to an agitation of 150 RPM, initial pH 6 and a temperature controlled at 23°C.

### Propagation

Following activation, the bacterial biomass is transferred to a flask with the medium of the present invention in the same stirring conditions, pH and temperature, to generate scalable inoculum cultures.

Once the strain of *P. salmonis* has been conditioned, we proceed to its spread into two defined media formulations, called MD1 and MD2, which are detailed in Table I. The minimum essential medium defined (MD) is made up by four groups of compounds: amino acids, salts, vitamins and minerals (micronutrients). The compounds are supplied individually. The formulation developed is detailed below in Table I.

**Table I: Specific composition of liquid culture media for Piscirickettsia salmonis bacteria. (MD) defined medium.**

| **Components** | **MD1g/L** | **MD2g/L** |
|---|---|---|
| ***Aminoacids*** | | |
| Glutamic acid | 0.645 | 1,925 |
| Threonine | 0.657 | 1,732 |
| Arginine | 0.645 | 1,185 |
| Lysine | 0.541 | 0,271 |
| Histidine | 0.56 | 0,14 |
| Cysteine | 1.01 | 0,336 |
| Phenylalanine | 0.405 | 0,101 |
| Valine | 0.517 | 0,129 |
| Leucine | 0.483 | 0,121 |
| Isoleucine | 0.483 | 0,121 |
| Methionine | 0.659 | 0,165 |

| ***Vitamins*** | | |
|---|---|---|
| Choline Chloride | 0.002 | 0.002 |
| D-calcium pantothenate | 0.002 | 0.002 |
| Folic acid | 0.002 | 0.002 |
| Niacinamide | 0.002 | 0.002 |
| Pyridoxal hydrochloride | 0.002 | 0.002 |
| Riboflavin | 0.0002 | 0.0002 |
| Thiamine hydrochloride | 0.002 | 0.002 |
| Inositol | 0.004 | 0.004 |

| ***Mineral salts*** | | |
|---|---|---|
| K₂HPO₄ | 6,3 | 6,3 |
| MgSO₄•7H₂O | 0,1 | 0,1 |
| NaCl | 9 | 9 |
| CaCl₂•2H₂O | 0,08 | 0,08 |
| Ferric citrate | 0,032 | 0,032 |
| KCL | 0,004 | 0,004 |
| Na HPO₄ | 0.023 | 0.023 |

The formulations shown in Table I as MD2and MD1 correspond to a specific detail that meets the specific nutritional needs of the bacteria of the genus *Piscirickettsia.* This was possible, since these formulations were obtained after the characterization of *P*. *salmonis* genomic and metabolic level. The results are unprecedented in specific literature for this organism.

Regarding the formulation of the medium MD1, this was designed to determine the essential nutrients (auxotrophies) required for growth of *P. salmonis* while MD2 formulation was designed after determining profiles specific nutritional demand over time, which allowed to adjust the concentrations of each component initially described in the medium MD1.

Culture medium MD1 and MD2 were developed for batch cultures. A third culture medium was also performed fed-batch (CLA) based on the medium MD2 (MD2-CLA).

The analyses permitted to determine that the carbon and energy sources required for growth of *P. salmonis* are glutamic acid, threonine and arginine. This information to date has not been reported in the literature.

In addition, the essential components of these media, such as histidine, cysteine, phenylalanine, valine, leucine, isoleucine, methionine and lysine, which as a whole are required for the related growth of protein synthesis and biomass determined in the study bacteria of the *Piriscirickettsia* genus. These amino acids are in the formulation described in patent application WO2013084169, but without distinguishing its essential condition, specificity and favorable amount for bacterial growth. All these aspects have become evident and determined in experimental assays of the present invention. This prevents unspecific use of other amino acids cited in WO2013084169, which are not used by the bacteria to increase its biomass. It is important to underscore this aspect, since the reduction in the number and amount of amino acids used, is not an obvious condition, since the absence of any of them at random can determine the inefficiency or delay in the biomass growth of the *Pisciricketsia* bacteria gender. However, the determination of specific amino acids allows the quick growth of the bacteria (in less time), efficiently and inexpensively.

In the literature (Mauel, Ware & Smith, 2008) it is indicated that the amino acid cysteine is an essential component for the growth of *P. salmonis.* However, this research has determined that seven other amino acids are essential and must be supplied to meet the auxotrophies allow the bacteria to grow.

The MD2 culture medium was adjusted in function of the key nutritional requirements of the Piscirickettsia bacterium genus, particularly the species *P. salmonis, this* adjustment was made according to the specific requirements of carbon and energy sources evidenced as being preferable. The concentration of the components that supplement metabolic deficiencies of the bacteria and of the micronutrients and mineral salts required for their growth was also adjusted.

In this formulation, other components are not added, such as carbohydrates, glycerol, lipids, non-essential amino acids, trace minerals, complex substrates (yeast extract, peptone, serum, blood) included in the formulations previously published for bacteria of the genus *Piscirickettsia* in particular *P. salmonis.*

In the present invention, it is shown that some of the substrates listed above for *P. salmonis* growth, are not assimilated by the bacteria, since it does not have the metabolic capacity means for use in cell free culture. It also found that other components described in the prior art do not supply the nutritional needs of *P. salmonis*, and are in some cases growth inhibitors.

The present invention provides a formulation that is more specific and simple in its composition than the culture media of the state of art. The specific culture medium of the present invention enables the generation of larger biomass yield per amount of nutrients in a shorter time (yield MD2 0.82 g/g; Table III, described below).

These results permit to mount a more economical industrial production line, including revolutionary characteristics in cultivating bacteria of the genus *Piscirickettsia,* particularly *P. salmonis.* Differences between media components described in the state of the art and the media MD1 and MD2 are compared in Table II.

**Table II. A comparison between cultivation means of the state of the art and MD1 and MD2**

| **Components** | **WO2013084169 g/L** | **WO2008002152 g/L** | **MD1 g/L** | **MD2 g/L** |
|---|---|---|---|---|
| **Inorganic Salts** | | | | |
| Ammonium nitrate | 0.0002 | ----- | ----- | ----- |
| Boric acid | 0.0028 | ----- | ----- | ----- |
| Calcium chloride (CaCl₂) (anhydrous) | 0.2461 | ----- | 0.08 | 0.08 |
| Disodium phosphate | 0.001 | ----- | ----- | ----- |
| Ferric citrate | 0.0126 | ----- | 0.032 | 0.032 |
| Ferric nitrate | 0.005 | ----- | ----- | ----- |
| Magnesium chloride | 0.7412 | ----- | ----- | ----- |
| Magnesium sulfate (MgSO₄) (anhydrous) | 0.4168 | 0.4 | 0.1 | 0.1 |
| Monobasic potassium phosphate (KH₂PO₄) | | 0.3 | 6.3 | 6.3 |
| Potassium bromide | 0.0101 | ----- | ----- | ----- |
| Potassium chloride | 0.1091 | 0.8 | ----- | ----- |
| Sodium bicarbonate (NaHCO₃) | 0.2401 | ----- | ----- | ----- |
| Sodium chloride (NaCl) | 7.1235 | 10 | 9 | 9 |
| Sodium fluoride | 0.0003 | ----- | ----- | ----- |
| Monobasic sodium phosphate (NaH₂PO₄-H₂O) | 0.014 | ----- | ----- | ----- |
| Sodium silicate | 0.0005 | ----- | ----- | ----- |
| Sodium sulfite | 0.2 | ----- | ----- | ----- |
| Strontium chloride | 0.0043 | ----- | ----- | ----- |

| **Other components** | | | | |
|---|---|---|---|---|
| D-glucose | 15.6 | ----- | ----- | ----- |
| Peptone N° 3 | 8.1281 | ----- | ----- | ----- |
| Pancreatic casein | 7.5 | ----- | ----- | ----- |
| Casein | | 5 | ----- | ----- |
| Soy peptone | 5 | ----- | ----- | ----- |
| Yeast extract | 0.3256 | 15 | ----- | ----- |
| Glycerol^{a} | | 10 | ----- | ----- |
| Saccharose^{a} | | 10 | ----- | ----- |
| Maltose^{a} | | 10 | ----- | ----- |
| FBS cell culture Gibco® (Fetal bovine serum) | 100ml | ----- | ----- | ----- |

| **Aminoacids** | | | | |
|---|---|---|---|---|
| Glycine | 0.0008 | ----- | ----- | ----- |
| L-alanine | 0.0009 | ----- | ----- | ----- |
| L-arginine | 0.0126 | ----- | 0.645 | 1,185 |
| L-asparagine-H₂O | 0.0013 | ----- | ----- | ----- |
| L-aspartic acid | 0.0013 | ----- | ----- | ----- |
| L-cysteine 2HCl | 1.7031 | ----- | 1.01 | 0,336 |
| L-glutamic acid | 0.0015 | ----- | 0.645 | 1,925 |
| L-histidine | 0.0042 | ----- | 0.56 | 0,14 |
| L-isoleucine | 0.0052 | ----- | 0.483 | 0,121 |
| L-leucine | 0.0052 | ----- | 0.483 | 0,121 |
| L-lysine | 0.0073 | ----- | 0.541 | 0,271 |
| L-methionine | 0.0015 | ----- | 0.659 | 0,165 |
| L-phenylalanine | 0.0032 | ----- | 0.405 | 0,101 |
| L-proline | 0.0012 | ----- | ----- | ----- |
| L-serine | 0.0011 | ----- | ----- | ----- |
| L-threonine | 0.0048 | ----- | 0.657 | 1,732 |
| L-tryptophan | 0.001 | ----- | ----- | ----- |
| L-tyrosine | 0.0052 | ----- | ----- | ----- |
| L-valine | 0.0046 | ----- | 0.517 | 0,129 |

| **Specific Vitamins** | | | | |
|---|---|---|---|---|
| Choline Chloride | 0.0001 | ----- | 0.002 | 0.002 |
| D-calcium pantothenate | 0.0001 | ----- | 0.002 | 0.002 |
| Folic acid | 0.0001 | ----- | 0.002 | 0.002 |
| Niacinamide | 0.0001 | ----- | 0.002 | 0.002 |
| Pyridoxal hydrochloride | 0.0001 | ----- | 0.002 | 0.002 |
| Riboflavin | <0.0001 | ----- | 0.0002 | 0.0002 |
| Thiamine hydrochloride | 0.0001 | ----- | 0.002 | 0.002 |
| Inositol | 0.0002 | ----- | 0.004 | 0.004 |

It is important to underscore that the cultivation medium of WO 2008002152, even when it has a few components, their composition is rich in undefined nutriaents (for example, yeast extract), which makes them non-specific. (^{a}) Separate evaluation of each substrate in patent (WO2008002152).

**Table III. A comparison of cultivation parameters by batch of P salmonis in formulated media MD1, MD2 and MD2-CLA with the state-of-the-art media.**

| | Maximum biomass per culture medium | | OD600nm to 60h culture | FC in culture medium (g/L) | Yx/s (OD/g FC) at tₘₐₓ | Qx/s (OD/h FC) at tₘₐₓ | Qx/s (OD/h) at 60h |
|---|---|---|---|---|---|---|---|
| | OD600nm | Time (h) | | | | | |
| MD1-batch | 2.25 | 60 | 2.25 | 7.1 | 0.32 | 0.04 | 0.04 |
| MD2-batch | 5.3 | 60 | 5.3 | 6.5 | 0.82 | 0.09 | 0.09 |
| MD2-CLA | 7.78 | 71 | 7.42 | 8.2 | 0.95 | 0.11 | 0.11 |
| WO2008002152 | 2.85 | 144 | <0.62 | 30 | 0.09 | 0.02 | 0.01 |
| SF900II* | 13 | 120 | 2 | | | 0.11 | 0.03 |
| WO2013084169 | 1.8 | 144 | 075 | 38.3 | 0.05 | 0.01 | 0.01 |

| | | | | | | | |
|---|---|---|---|---|---|---|---|
| (Yx/s) The substrate yield in bacterial biomass indicates the biomass produced per gram of substrate. (Qx/ s) Productivity, indicates the biomass produced per hour (*) SF900II™ is a commercial means for cell lines of *Spodoptera frugiperda* supplied by Life Technologies, reported in WO2008002152 to isolate and evaluate pH conditions for cultivating *P. Salmonis.* (FC) Total carbon source. (OD) Optical density. (tmax). Cultivation time where the OD maximus is reached. | | | | | | | |

The formulation for culture media MD2 and MD2-CLA of the invention, delivering indices culture are significantly better than those reported in the prior state-of-the-art (Yx/s: performance substrate bacterial biomass; Qx/s: productivity). Performance for each culture medium of the invention was calculated by measuring the optical density (OD) maximum per gram of the compounds that are used by the bacteria as carbon source potential. All compounds which can be assimilated by the bacteria as a carbon source for this calculation were considered, since these culture media were designed without being clear which compounds were metabolized by *P. salmonis.*

The productivity was calculated as the ratio between the OD of the culture and the time it took to reach that OD. At 60 h, the medium MD2 provided a productivity of 0.09 OD / h, while WO2008002152 and WO2013084169 reported 0.01 OD/h. Similarly, MD2 and MD2-CLA showed superior performance, reaching 0.82 OD/g and 0.95 OD/g respectively of carbon source (FC), which contrasts with the poor performance achieved by WO2008002152 and WO2013084169 which is less than 0.1 OD/g FC. SF900II™ commercial medium was used in WO2008002152 to isolate and evaluate different culture conditions *P. salmonis*, where good growth rates were reported. While the formulation of this medium was not found available, you can get a productivity index which reaches 0.11 OD/h, slightly higher than in MD2 and the same for the case of MD2-CLA. However, when comparing productivity obtained at 60h of culture, it can be seen that MD1, MD2 and MD2-CLA D2 and all of them outperformed all media reported and evaluated (Table III).

The MD1, MD2 and MD2-CLA media significantly improve cultivation rates being these in less time than the average reported in the prior state of the art.

Advantageously, Table III shows that the amount of compounds that are a source of carbon and energy (FC & E) in MD1, MD2 and MD2-CL are considerably less than the media reported.

The MD2-CLA has a productivity (Qx/s) significantly higher than all other culture media at 60 h. SF900II medium shows only equivalent productivity in time where maximum biomass is reached. D2 exhibits high productivity under lower cultivation times and maintained until the end of culture.

Table III shows that the improvement in the specific amounts of nutrients and general specificity of the culture media formulated enable higher biomass in less time compared to unspecific culture media indicated in the state of the art WO2008002152 and WO2013084169. Both MD2 as Md2- CLA show better productivity rates at 60h culture compared to commercial medium SF900II™ (Table III). Consider that this commercial medium is formulated for the proliferation of cell lines, therefore it contains unnecessary components in its formulation for *P. salmonis*; along with costs higher than the estimated value of MD2 and MD2-CLA. The price of the culture medium for fermentation processes is critical. Therefore, a process using MD2 or MD2-CLA instead of SF900II shows very important advantages in terms of cost (cost SF900II is app 7.4 times higher; SF900II™ costs approximately CL $ 63,000 per liter; MD2 costs approximately CL $ 8,500 per liter).

The culture medium SF900II is a trademark of unknown composition for the public therefore the composition of MD2 and MD2-CLA are not derived from the composition of this brand. Additionally, the MD2 and MD2-CLA are minimally defined culture media specially designed for the growth of *P. salmonis.*

The availability of the culture medium SF900II depends on the provider, because the provider may decide not to produce the product anymore; instead D2 and MD2-CLA culture media do not depend on one supplier alone.

The state of the art reports the growth of *P. salmonis* in OD as a measure to indicate biomass production levels achieved in each crop. The present invention, also reports bacterial growth as dry weight biomass concentration (g dry biomass / L) or as a number of bacteria (N° bacteria/mL).

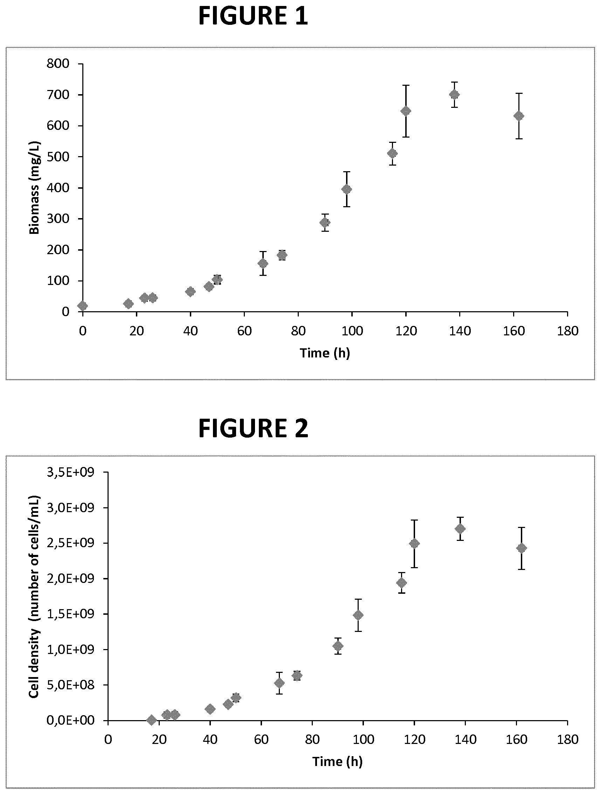

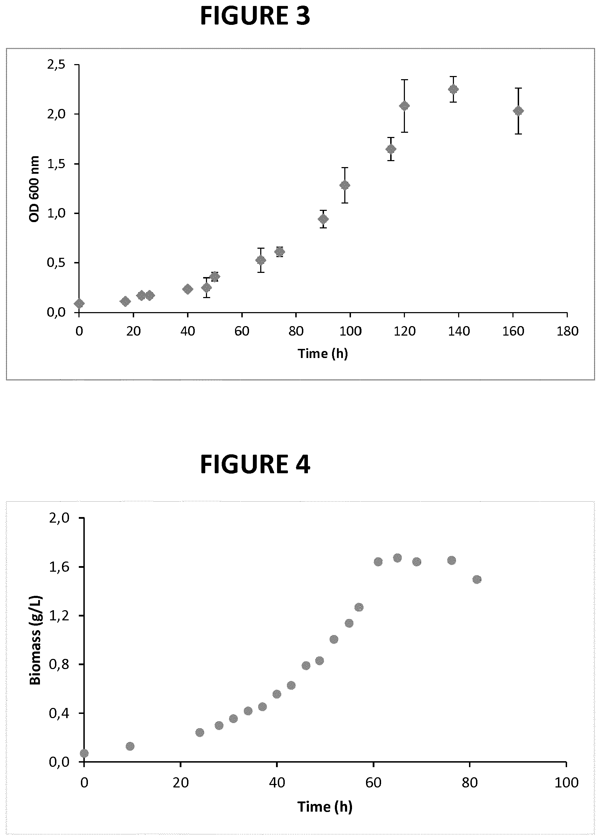

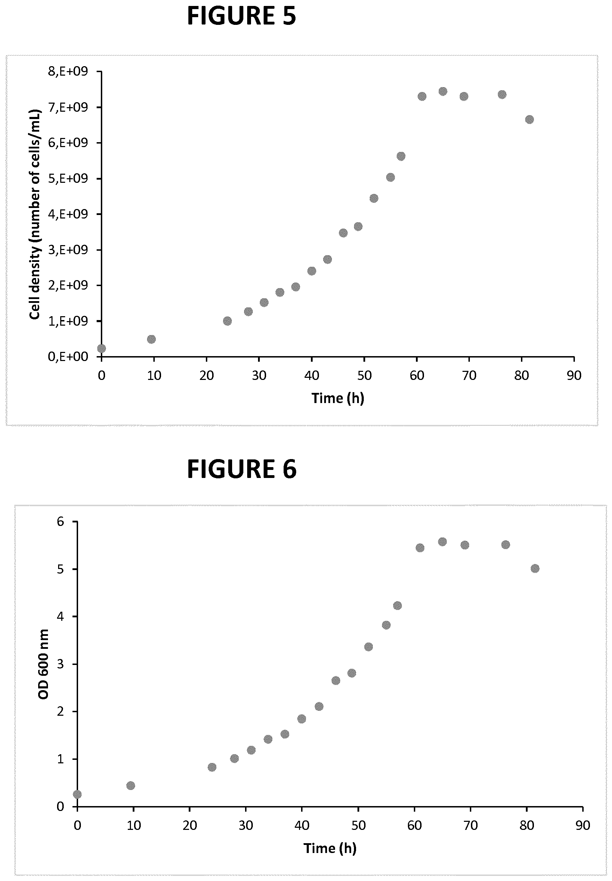

Figures 1 to 6 show the growth kinetics of *P. salmonis* documented for media MD1 and MD2, where the time to reach maximum biomass production is achieved around 60 h (2.5 days), with an average inoculum close to 90 ± 10 mg/L at 0 h. Considerably more efficient than data published in the state of the art. Average biomass production reached 0.75 ± 0.05 g/L (equivalent to 2,7x10⁹ N° bacteria/mL, 2.25 OD) in MD1 (Figure 1, 2 and 3) and 1, 5 ± 0,1 g/L (equivalent to 7,3x10⁹ N° bacteria/ml; 5.5 OD) in MD2 (Figure 4, 5 and 6), while specific growth rate (µ) of *P. salmonis* in MD1 is 0.03 ± 0.005 h⁻¹ and the MD2 is 0.045 ± 0.005h⁻¹.

The formulation of a specific liquid culture medium defined for growth of *P*. *salmonis* permits to avoid host cells and therefore avoid the complications derived from this type of culture (culture of host cell, infection of the host cell, separation of bacterial biomass from eukaryotic cells) and also allows a biomass free of contamination (the host cell structures). Culture media MD1 and MD2 and have defined the ratio and quality of its components, which standardizes and certifies each batch of medium used for growing *P. salmonis.* This is an advantage for the industry, since by controlling the composition of the culture medium in a production line, it permits to replicate the parameters of productivity and culture yield in time. This is possible in the MD1 and MD2 media, because they do not use nonspecific complex substrates such as: peptone, yeast extract or serum, which component proportions can vary among batches and providers, making it difficult to replicate and hence to forecast productivity and performance for a production line.

Additionally, MD1 and MD2 components show a standard quality, which enables the industry to change suppliers without taking risks at a manufacturing level. This new formulation provides the bacteria only with basic and essential components that allow the proliferation of *P. salmonis,* reducing or eliminating compounds the bacteria do not use and turning the production of bacteria into a more profitable business compared to the formulation of a complex environment with many components (Solomons, G. L, (1969) Materials and Methods in Fermentation; Academic Press. New York, 133-149). The generation of culture media MD1 and MD2 based on specific nutritional requirements of the bacteria analyzed specifically according to experimental conditions of every testing procedure and therefore following a specific design which covers their nutritional requirements.

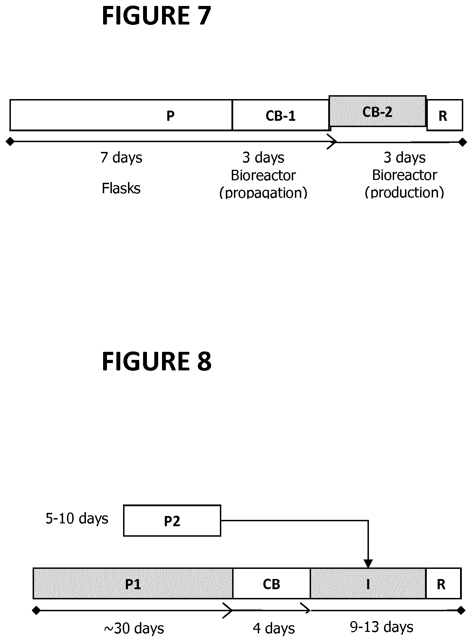

Cultivation of *P. salmonis* evaluated at a bioreactor scale was first described herein. The inventors successfully scaled to pilot scale cultures of *P. salmonis* in MD-2, reproducing the biomass concentration rates(OD₆₀₀), yield (Yx/s) and productivity (Qx/s) reported in lower volumes (see Tables IV and V).
As mentioned above, cultivation takes place in stages such as: dissemination, culture in bioreactor and recovery. The dissemination involves the successive culture of bacteria in flasks with increasing volumes of culture medium (MD-2), and the generation of the inoculum, in the bioreactor, to initiate the culture process at pilot scale (the volume of inoculum corresponds to 10% the volume of production at a pilot level). The stage lasts 10 days. The stage in the bioreactor at pilot scale includes the vulture of bacteria in a defined MD-2 medium under controlled conditions of temperature, pH, dissolved oxygen concentration and stirring rate. This stage lasts 3 days. The stage of recovery is performed by centrifugation in one step which produces pure biomass of *P. salmonis.* The duration of this stage is negligible compared to the total time. The process thus described has an overall duration of 13 days (Figure 7).

Currently *P. salmonis* biomass required for the formulation of vaccines is produced by infecting eukaryotic cell lines. Unlike the description in the preceding paragraph, the process used hitherto for the cultivation of *P. salmonis* involves five stages: 1. Propagation of the cell line; 2. Dissemination of *P. salmonis* in the cell line; 3. Culture of cell line in the bioreactor; 4. Infection of cell line cultured in the bioreactor with *P. salmonis;* 5. Recovery of *P. Salmonis* through centrifugation. This last operation is performed through two centrifugation steps. It should be noted that biomass so harvested can contain traces of the eukaryotic cell line that can adversely affect the level of protection the vaccine can provide.

Figure 8 shows an overview of this process indicating all involved times. The overall process lasts 43 to 47 days. The costs this system involves are high, due to the time of production cycles and the costs associated to services, human resources and operational inputs. This last item is critical since inputs (culture media, operational consumables) needed for the propagation of cell lines are highly expensive. The market value per liter of culture medium for the cell line where *P. salmonis* is disseminated costs over CL$ 60,0 00. Moreover, the propagation of the cell line involves the replacement of the culture medium every 2 to 3 days. It is also worth to mention that systems where the cell line is cultivated as disposable, with reduced workloads and high costs, since they are special materials that must not affect the viability of eukaryotic cells. Such operational costs increased significantly due to the prolonged time of the process, in addition to the number of skilled workers required for the maintenance of cultures.

### Examples:

The examples described below refer to a practical demonstration of the advantages associated to the culture medium of the present invention.

### Example 1: Cultivation of P. salmonis in batch mode at a pilot scale (10L bioreactor culture) in culture medium MD-2.

Among defined media formulations, MD-2 is the one generating higher levels of biomass using the same culture times. These cultures were developed at a controlled temperature of 23°C, with a stirring rate of 150 rpm, 1 vvm aeration and a pH of 6.0. Batch culture mode was scaled up to a 10 L volume, the pilot scale for this system. The results obtained are shown in Table I. Table IV sows the use of MD-2 culture medium at different scales, from a flask to a 2, 5 and 10 L bioreactor. The 10L scale corresponds to a pilot scale for this type of process.

**Table IV. Culture media and systems implemented for the cultivation of P. salmonis**

| | | | | Culture | | | | |
|---|---|---|---|---|---|---|---|---|
| Medium | Mode | Scale | Volume (L) | Time (h) | OD₆₀₀ₙₘ | FC in culture medium (g/L) | Yx/s (OD₆₀₀ₙₘ/g FC) | Qx/s (OD₆₀₀ₙₘ/h) at 60h |
| MD-1 | Batch | flask | ≤0,3 | 60 | 2,25 | 7,1 | 0,32 | 0,04 |
| MD-2 | Batch | Flask | ≤0,3 | 60 | 5,3 | 6,5 | 0,82 | 0,09 |
| MD-2 | Batch | bioreactor | 1,5 and 10L | 60 | 5,3 | 6,5 | 0,82 | 0,09 |
| MD-2 | Fed Batch | bioreactor | 2 | 60 | 7,4 | 8,2 | 0,95 | 0,11 |

| | | | | | | | | |
|---|---|---|---|---|---|---|---|---|
| Operational indexes. Yx/s: Performance of substrate in bacterial biomass. Qx/s: Productivity. FC: Source of total aggregate carbon. OD600nm: optical density at 600 nm. | | | | | | | | |

Table IV shows that batch mode in MD-2 can be reproduced at different volumes maintaining the biomass concentration rates, performance and productivity both at flask and bioreactor scale. Moreover, this cultivation can increase biomass 19 times compared to the average initial concentration of 0.28. Table V (described below).

**Table V. Comparison of operational conditions of cultures of P. salmonis in the media mentioned in the current invention and the ones reported in the previous state of the art.**

| | Operational conditions | | | | |
|---|---|---|---|---|---|
| | Culture system | Stirring (rpm) | Temperature (°C) | Inoculum (OD₆₀₀ₙₘ) | pH |
| MD1 | Flask | 100 | 23 | 0.28±0.03 | 6.0^{a} |
| MD2 | Flask and reactor by batch | 150 | 23 | 0.28±0.03 | 6.5_{b} |
| MD2 | Reactor by fed batch | 150 | 23 | 0.28±0.03 | |
| WO2008002152 | Flask | 100 | 20 | NR | 6.2^{a} |
| SF900II* | Flask | 75 | 20 | NR | 6.5^{a} |
| WO2013084169 | Flask | 50 | 18 | 0.25 | |

| | | | | | |
|---|---|---|---|---|---|
| NR: Not reported ^{(a)} free pH cultures, being the pH reported the initial one. ^{(b)} Initial pH in flask and controlled in the reactor. (*) SF900II™ is a commercial medium for *frugiperda Spodoptera* cell lines supplied by Life Technologies, reported in WO2008002152 to isolate and evaluate pH conditions for the culture of *P. salmonis.* | | | | | |

### Example 2: Culture of P. salmonis in 2L bioreactor fed batch mode in culture medium MD-2 (MD2-CLA).

Fed-batch cultivation was another strategy for the fermentation of P. salmonis, with which higher levels of biomass can be reached. This strategy was tested under the same operating conditions as batch cultures. The difference lies in the controlled reception of feeding of culture medium based on MD-2. The results obtained are shown in Table IV and demonstrate that this strategy yields higher biomass concentrations, higher yields and productivity than the one obtained previously.

### Example 3: Infectivity of P. salmonis cultured in MD-2.

*P. salmonis* infectivity testing showed the conservation of a virulent phenotype in cultures in MD-2. Testing was performed under a controlled environment, with daily supervision and daily monitoring of the parameters of water temperature and oxygen saturation. It was tested with 180 fish of the species *Salmo salar*, with an average weight of 30 grams, in pre-smolt stage.

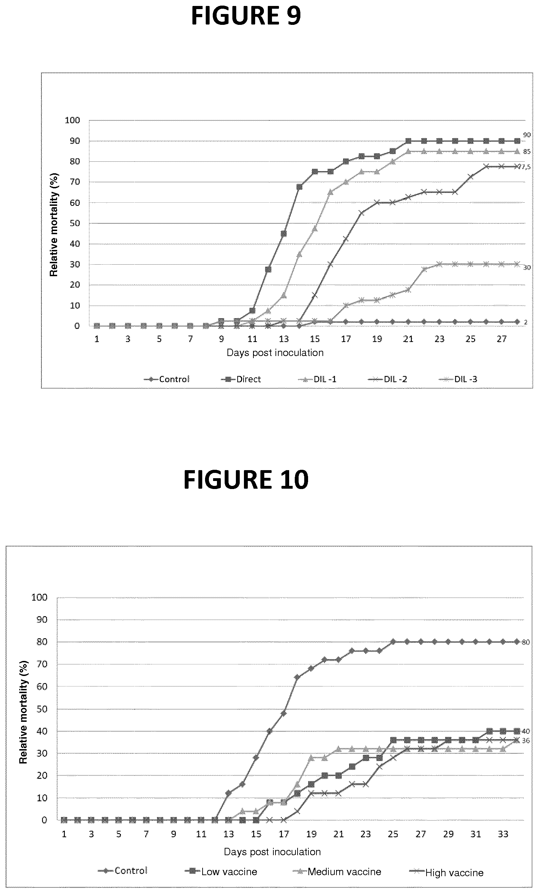

All fish challenged with *P. salmonis* showed signs and lesions described in cases of SRS. Time to the start of mortality was also the expected for this species, strain and the conditions used in this study. Fish were challenged with 4 concentrations of *P*. *salmonis* being Direct the highest concentration and D1, D2 and D3 a serial dilution of it, different in one logarithm of concentration and measured in No. of bacteria/mL. Mortalities obtained in this assay (Figure 9) lead us to calculate DL50. Fatality rate calculated for *P. salmonis* cultured in MD-2, reached the level of virulence of environmental strains. Therefore, cultures in MD-2 permit to preserve the virulent phenotype observed in environmental strains.

### Example 4: protection index of vaccines made from biomass from crops MD-2.

Vaccines have been developed with the biomass obtained from cultures in MD-2, which have showed improvements in protection rates compared to traditional vaccines manufactured with *P. salmonis* biomass obtained from cultures of eukaryotic cell lines. The biomass obtained from cultures in MD-2 was inactivated by using a chemical method for the manufacture of vaccines. In this test, vaccines were developed with high, medium and low antigen load being the high load 3 times more concentrated than the medium load and the medium load 3 times more concentrated than the low load. Subsequently, fish of species *Salmo salar* 70 g, were challenged with the dose defined in DL50. The results show that regardless of their concentration, the vaccines produce the same level of protection in relation to the control (unvaccinated fish; Figure 10).

## Claims

1. A chemically defined liquid culture medium which formula based on the minimal composition to meet the specific nutritional requirements of Piscirickettsia bacteria, especially Piscirickettsia salmonis, involving three groups of chemically defined components.

2. The chemically defined liquid culture medium defined in claim 1, CHARACTERIZED because it includes the following chemically defined components:
| **Components** | **MD1g/L** | **MD2g/L** |
|---|---|---|
| ***Aminoacids*** | | |
| Glutamic acid | 0.645 | 1,925 |
| Threonine | 0.657 | 1,732 |
| Arginine | 0.645 | 1,185 |
| Lysine | 0.541 | 0,271 |
| Histidine | 0.56 | 0,14 |
| Cysteine | 1.01 | 0,336 |
| Phenylalanine | 0.405 | 0,101 |
| Valine | 0.517 | 0,129 |
| Leucine | 0.483 | 0,121 |
| Isoleucine | 0.483 | 0,121 |
| Methionine | 0.659 | 0,165 |
| ***Vitamins*** | | |
|---|---|---|
| Choline Chloride | 0.002 | 0.002 |
| D-calcium pantothenate | 0.002 | 0.002 |
| Folic acid | 0.002 | 0.002 |
| Niacinamide | 0.002 | 0.002 |
| Pyridoxal hydrochloride | 0.002 | 0.002 |
| Riboflavin | 0.0002 | 0.0002 |
| Thiamine hydrochloride | 0.002 | 0.002 |
| Inositol | 0.004 | 0.004 |
| ***Mineral salts*** | | |
|---|---|---|
| K₂HPO₄ | 6,3 | 6,3 |
| MgSO₄•7H₂O | 0,1 | 0,1 |
| NaCl | 9 | 9 |
| CaCl₂•2H₂O | 0,08 | 0,08 |
| Ferric citrate | 0,032 | 0,032 |
| KCL | 0,004 | 0,004 |
| Na HPO₄ | 0.023 | 0.023 |

3. A scalable process in the bioreactor to cultivate Psicirickettsia bacteria, especially Piscirickettsia salmonis, in a chemically defined liquid medium.

4. The process for the cultivation bacteria in claim 3, CHARACTERIZED because it produces the highest volumetric yield in the lowest time.

5. A method of growing bacteria of the genus piscirickettsia, in particular of piscirickettsia salmonis in a chemically defined liquid medium of claim 1, **CHARACTERIZED in that** a chemically defined culture medium scalable in bioreactor is inoculated.

## Amended claims

### Amended claims under Art. 19.1 PCT

**1.** A minimal liquid culture medium essential for the culture of Piscirickettsia gender bacteria, especially Piscirickettsia salmonis, including three groups of constituents: aminoacids, mineral sales and vitamins as micronutrients.

**2.** The liquid culture medium of claim 1 includes the following components:
| **Components** | **MD1g/L** | **MD2g/L** |
|---|---|---|
| ***Aminoacids*** | | |
| Glutamic acid | 0.645 | 1,925 |
| Threonine | 0.657 | 1,732 |
| Arginine | 0.645 | 1,185 |
| Lysine | 0.541 | 0,271 |
| Histidine | 0.56 | 0,14 |
| Cysteine | 1.01 | 0,336 |
| Phenylalanine | 0.405 | 0,101 |
| Valine | 0.517 | 0,129 |
| Leucine | 0.483 | 0,121 |
| Isoleucine | 0.483 | 0,121 |
| Methionine | 0.659 | 0,165 |
| ***Vitamins*** | | |
|---|---|---|
| Choline Chloride | 0.002 | 0.002 |
| D-calcium pantothenate | 0.002 | 0.002 |
| Folic acid | 0.002 | 0.002 |
| Niacinamide | 0.002 | 0.002 |
| Pyridoxal hydrochloride | 0.002 | 0.002 |
| Riboflavin | 0.0002 | 0.0002 |
| Thiamine hydrochloride | 0.002 | 0.002 |
| Inositol | 0.004 | 0.004 |
| ***Mineral salts*** | | |
|---|---|---|
| K₂HPO₄ | 6,3 | 6,3 |
| MgSO₄•7H₂O | 0,1 | 0,1 |
| NaCl | 9 | 9 |
| CaCl₂•2H₂O | 0,08 | 0,08 |
| Ferric citrate | 0,032 | 0,032 |
| KCL | 0,004 | 0,004 |
| Na HPO₄ | 0.023 | 0.023 |

**3.** A process to cultivate Piscirickettsia bacteria, especially Piscirickettsia salmonis in extracellular liquid medium mentioned in claim 1.

**4.** The process for the cultivation of bacteria in claim 3 with maximal concentration of bacteria reached in a 3 to 4 days period.

**5.** A method to grow Piscirickettsia bacteria, especially Piscirickettsia salmonis in extracelular liquid medium in claim 1, where the bacteria is inoculated in a culture medium both in a flask or a bioreactor.
